(19) Europäisches Patentamt / European Patent Office / Office européen des brevets

(11) EP 4 530 907 A1

(12) **EUROPEAN PATENT APPLICATION**

(43) Date of publication:
02.04.2025 Bulletin 2025/14

(21) Application number: 23200974.6

(22) Date of filing: 29.09.2023

(51) International Patent Classification (IPC):
**G06F 21/62** (2013.01)     **G06N 3/0455** (2023.01)
**G06N 3/0464** (2023.01)     **G16H 10/60** (2018.01)
**G16H 30/00** (2018.01)     **G06N 3/084** (2023.01)
**G06N 3/094** (2023.01)

(52) Cooperative Patent Classification (CPC):
**G06F 21/6254; G06N 3/084; G06N 3/094;**
**G16H 10/60; G16H 30/00;** G06N 3/0455;
G06N 3/0464

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: Koninklijke Philips N.V.
**5656 AG Eindhoven (NL)**

(72) Inventors:
• **SAALBACH, Axel**
  **Eindhoven (NL)**
• **NICKISCH, Hannes**
  **Eindhoven (NL)**

(74) Representative: **Philips Intellectual Property &**
**Standards**
**High Tech Campus 52**
**5656 AG Eindhoven (NL)**

(54) **DEFENDING AGAINST PATIENT RE-IDENTIFICATION ATTACKS**

(57) System (SYS) and related method for processing of data. The system may comprise an interface (IN) capable to receive input data based on personal data processable by a target machine learning model (M) for producing a result. A modifier (MOD) of the system is capable of modifying the input data into modified input data, such that the said result corresponds to a result obtainable by having the modified input data processed by the target machine learning model (M), whilst the modified data is still capable of defending against a re-identification attack.

FIG. 2

**Description**

FIELD OF THE INVENTION

The invention relates to a system for processing of data, to a related arrangement and method, to a computer program element, and to a computer readable medium.

BACKGROUND OF THE INVENTION

Person re-identification in camera images or video streams is an active field of research with many applications, specifically in the security domain.
Recently, such techniques have been successfully employed to chest X-Ray images, such as reported by K Packhäuser et al in "Deep learning-based patient re-identification is able to exploit the biometric nature of medical chest X-ray data", published in NATURE, scientific reports. Vol 12: 14851, 2022. The authors utilized a Siamese Neural Network (SNN) with Contrastive Loss to generate a low-dimensional mapping of the input data. Using this setup, the network learns to map images from the same patient to nearby points in the low-dimensional space, while images from different patients are mapped to distant points. Using different datasets, they were able to consistently achieve very high re-identification scores (Precision@1 between 0.882 to 0.996).
A more detailed analysis further indicates a strong robustness with respect to the presence of abnormalities and the time span between the acquisitions. These results indicate that common deidentification techniques for medical image (covering DICOM tags or burned in annotations), might not be sufficient to protect the identity of a patient.

SUMMARY OF THE INVENTION

There may therefore be a need for improved methods and systems for defending against re-identification attacks, in particular in respect of personal data, such as medical or other.
An object of the present invention is achieved by the subject matter of the independent claims where further embodiments are incorporated in the dependent claims. It should be noted that the following described aspect of the invention equally applies to the related arrangement and method, to the computer program element and to the computer readable medium.
According to a first aspect of the invention there is provided a system for processing of data, comprising:

an interface capable to receive input data based on personal data processable by a target machine learning model for producing a result; and
a modifier capable of modifying the input data into modified input data, such that the said result corresponds to a result obtainable by having the modified input data processed by the target machine learning model, whilst the modified data is still capable of defending against a re-identification ("re-ID ") attack.

The data may describe one of more aspects of an individual (a person).
The said personal data may include patient data for example, such a medical imagery.
In particular the said correspondence is such that the target model as applied to the (original) input data yields a result that is equal to the said result obtainable by having the modified input data processed by the target machine learning model. In addition, and as a distinction, a defense against the re-ID attack is such, that responses of the re-ID attack are different for the original data than it for the modified data. In particular, a further/different, ML model ("adverse model") may be used to implement the re-ID attack. In such scenario, a result of applying the adverse model to the original input data is different from a result obtainable when applying the adverse mode to the modified data. Thus, the input data is so modified herein by the proposed system that only partial invariance is achieved with respect the two models, the target and the adverse model.
The modified data may at least reduce likelihood for a given re-ID attack to succeed, as compared to when processing the original data in the re-ID attack.
In embodiments, the modifier is capable of so modifying the input data based on the gradient of the target model.
*"Gradient"* as used herein is a way/computational device/algorithm for quantifying rate of change of the model, with respect to the model's input data. Model may be represented as a predictor or estimator function and the gradient of the model is the gradient of said function. Gradient can be computed by using numerical techniques, with or without discretization.
In embodiments, the modifier is capable of so modifying along a direction other than a direction of the said gradient of the target model.
The data may be represented, eg by embedding, as elements of a suitably structured data space, such as a vector space, preferably with a defined concept of distance (function) or similarity measure/function, such as a normed vector space, such as a Euclidean vector space, or merely a vector space with a metric. The said direction may thus be defined in said space, such as in said vector space. The modification operation by modifier may be implemented by application of a modifier vector to the input data, wherein each, the data, the modifier vector and the modified data are elements in said data space. The modifier vector may be the computed/determined by modifier based on said model's gradient at the considered data (point/vector).

In embodiments, the modifier is capable of so modifying along a direction that is essentially perpendicular to the direction of the said gradient of model.

As mentioned above, in embodiments, the re-identification attack is performable by the further (adverse) machine leaning model.

In embodiments, the modification is (further) based on a gradient of said further model.

In embodiments, the modification is based on a gradient of the distance function, where such distance function measures the distance between the results obtainable by the further machine leaning model.

In embodiments, the said result corresponds to a domain task.

In embodiments, the said task is medical.

In embodiments, the task is based on any one of a regression or classification and such is performed by the target model. Such task may include semantic segmentation, instance segmentation or detection/localization, either of which may be conceptualized as classification or regression.

The operation of the further model is a re-ID attempt (attack) which may be conceptualized as a classification task. The classification task, if any, performable by the target model is different from the classification task of further mode (adverse model). The classification as per target model is a domain task, such as disease detection based on input patient imagery, or other such as in medical domain. Thus, target model is not configured for re-ID purpose, whilst adverse model is so configured.

In embodiments, the at least one of the target model or the further model is based on an artificial neural network.

In embodiments, the artificial neural network has multiple hidden layers.

In embodiments, the personal data includes patient data, such as medical imagery, lab tests, reports, etc. Medical imagery (of internals of different respective patients) is of main interest herein.

Thus, in embodiments, the personal data includes image data. Such image data may include still imagery, or video. It may be 2D. 3D or 4D, with time being one of the dimensions in the 4D=(3D.t) imagery, and, optionally, in the 3D = (2D,t) imagery, besides the 2 or 3 spatial dimensions. Alternatively, the 3D is fully spatial, such as a CT (computed tomography) volume.

In another aspect there is provided an arrangement including the said system of any one of the above described aspect or embodiment, and one or more of: i) one or more memories on which is stored the input data, ii) the target model, iii) the further model, iv) a data representation transformer capable of providing the input data based on the personal data.

In yet another aspect there is provided a method of data processing, comprising:

receiving input data based on personal data, processable by a target machine learning model for producing a result; and

modifying the input data into modified input data, such that the said result corresponds to a result obtainable by having the modified input data processed by the target machine learning model, whilst the modified data is still capable of defending against a re-identification attack.

In yet another aspect there is provided at least one computer program element, which, when being executed by at least one processing system, is adapted to cause the processing system to perform the said method.

In yet another aspect there is provided at least one computer readable medium having stored thereon the at least one program element, and/or on which is stored the target model or the further model, or an approximation of the said further model.

Thus, what is proposed herein is a novel system and method to defend against image-based re-identification attacks. The method is designed to alter the original image so that risk of a Deep Learning-based re-identification is minimized while the response of the target model, such as diagnostic Deep Learning algorithm/model, is preserved. Thereby the approach is not restricted to X-Ray images but could be employed to data from other modalities as well.

With the proposed modification, the results of the target model are the same for both, the original data and the modified data. However, the modification also ensures that with high likelihood the processing by the attacker of the modified data yield different results as compared to a processing of the original data. Thus, the proposed modification amounts to a scrambling having a "partial invariance property": veracity of results are preserved for the target model, but compromised for the adverse re-ID model of a re-ID attacker.

The proposed technology could be integrated into data storage systems (e.g., PACS) to enhance existing anonymization / de-identification functionality.

The proposed image-based de-identification techniques could be employed for data acquired in the context of research / product development activities in order ensure higher data protection standards.

Application of the proposed system and method in the medical domain is mainly envisage herein. That being said, again, such medical applications, whilst mainly envisaged, are not at the exclusion of other, non-medical applications also envisaged herein in alternative embodiments.

In general, the term *"machine learning"* includes a computerized arrangement (or module) that implements a machine learning ("ML") algorithm. Some such ML algorithms operate to adjust a machine learning model that is configured to perform ("learn") a task. Other ML operate direct on training data, not necessarily using such as model. This adjusting or updating of the model is called "training". In general task performance by the ML module may improve measurably, with training experience. Training experience may include suitable training data and exposure of the model to such data. Task performance may improve the better the data represents the task to be learned. *"Training experience helps improve performance if the training data well represents a distribution of examples over which the final system performance is measured"*. The performance may be measured by objective tests based on output produced by the module in response to feeding the module with test data. The performance may be defined in terms of a certain error rate to be achieved for the given test data. See for example, T. M. Mitchell, "Machine Learning", page 2, section 1.1, page 6 1.2.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 shows a block diagram of a data processing system;
Fig. 2 shows a block diagram of a data processing component configured for defending against a re-identification attack;
Fig. 3 illustrates operation of the re-identification defender component in Fig. 2 according to one embodiment; and
Fig. 4 shows a flow chart of a computer implemented method for defending against re-identification attacks.

DETAILED DESCRIPTION OF EMBODIMENTS

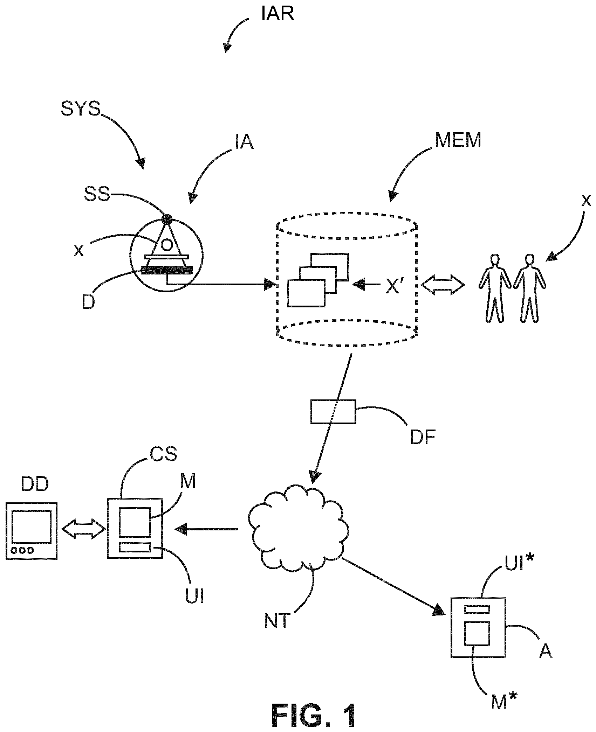

Reference is now made to the block diagram of Fig. 1 which illustrates components of a data processing system SYS. The system SYS may process data *X'*. Data *X* may be held in one or more data memories MEM. The data *X'* may be personal data pertaining to one or more individuals *x*. Thus, the data A may be personal data such as medical data as held in one or more databases at medical facilities, hospitals, GP practices etc. Indeed, operation of the data processing system SYS as envisaged herein in embodiments will be explained with main reference to the medical domain with the understanding that other (non-medical) applications are not excluded herein. Broadly, the system is configured with a defense mechanism DF against abuse of data, such as in one or more re-identification attacks that may be perpetrated by one (or more) attacker(s).

In such re-identification attacks, the attacker may use computational techniques one or more computer systems A (which will also be referred to herein as the "attacker (system)". The system A may use computing resources (such as run a software) any may be desirable to process the data X' in order to determine the individual (the person) to which the data X' belongs or otherwise pertains. The data X' may be accessible via one or more computing networks NT which may be wired, wireless or hybrid as required. The system may be arranged as shown in cloud setting where the data is held in database or others one or more storages, remote from the attacker A and/or a legitimate user CS of such data.

Thus, in addition to attacker A, there may be a legitimate data consumer CS, such as a computer system operable by a medical practitioner or medical professional, in order to process a patient's data X . Whilst the systems A, CS are shown as separate computing entities, such is not necessarily so in all embodiments. Both may be the very same system, but used at different times or simultaneously by different users, legitimate user and attacker, that each run different software or code for different purposes, for example such as a computing system in a pay-for-access computing center, such as an "internet café" establishment, etc. Also, the designation "legitimate" user may or may not imply that such user is authorized (eg, has valid user credentials) to access the data X', although in most cases such user CS will indeed be so authorized. What matters for present purposes herein is that the legitimate user CS does preferably not process the data for re-ID, whilst the attacker system A does. The attacker system may or may not be authorized for data access. Attacker may penetrate the system instead to gain access by "hacking" their way into data storage MEM. Also, as user herein the term "attacker" may or may not imply malicious intent. In some cases, such intent may not as such be malicious , or when an attacker merely seeks to obtain re-ID information for commercial or research purposes, of example. In other cases, sadly, intention may indeed be malicious, such as in obtaining information for blackmailing or other. However, malicious or not, what matters herein for present purposes is that there is personal data and information derivable therefrom which the data owner (e.g., patient or other entity who holds the data) may not wish to divulge or have disclosed, either explicitly or implicitly, for whichever reason.

Relating now, for the sake of example and definiteness, to the medical domain in more detail, the said data X' may relate to medical records. The data X' may include for example medical imagery. Medical imagery can be understood as measurements in respect of a given induvial patient x, obtained or acquired by a data source IA, such as an imaging apparatus. Imaging apparatus may include a signal source SS that is operable to emit signals to interact with patient tissue. The resulting interaction event is detected by a detector device D as the said measurements to obtain the imagery X'. Such medical imagery so measured represents internal aspects of patient, such as structure of internal tissues, organs, etc, in distinction to optical imagery of a patient's face, outer body (parts), etc. Such medical imagery X' may be stored in order to support therapy and/or diagnosis. Examples of such imagery envisaged herein as personal data X' include X-ray, radiographs, such as chest X-rays, CT imagery, other tomographic imageries such PECT, MRI, Ultrasound or any other.

Generally, the said X' data is personal data in that it pertains to a respective individual x. Thus, the data is personal as it may allow establishing aspects of a respective patient or individual x to which the data, such as the said image measurements, pertains. In extreme cases, such personal data may allow even unique identification of the respective individual. The processing of data with the aim for such identification may be called re-identification ("re-ID") attack. Anonymizing techniques have been used in the past to prevent re-identification attacks which may be undesirable as the patient may not want to have certain personal information divulged to parties A, CS. Some data, although personal, may in fact not allow unique identification of the patients. Such data are sometimes referred to as quasi-identifier, in distinction to other data items, called identifiers, that do allow such unique identification. For example, such identifier data may include a person's passport number, or other ID number etc. The place (a city for example) of residence, or place of birth, on the other hand, on its own, is usually considered a quasi-identifier as it does not allow unique identification of the individual x. Medical imagery, as opposed to optical imagery of a face for example, has been previously thought of as a quasi-identifier as it was thought for example that a patient's chest X-ray as such does not allow the unique identification of the patient. Recent tests with machine learning model-based processing have shown this not to be true (see for example the *Packhäuser* paper cited above). Thus, it has been shown that an individual x may well be identified by using a suitably configured machine learning ("ML") model to process such medical imagery X'. De-identification models may allow computing a probability that a certain medical image (X-ray, CT, MRI etc) belongs to a certain individual patient. Thus, such techniques may allow the attacker to establish for example whether a patient has in fact visited a certain medical facility when processing the data X' of the facility as held in data memory MEM. Such re-ID attack may be undesirable. Thus, in order to avoid, or at least reduce the likelihood of such re-identification attacks succeeding, the system SYS as proposed herein includes a re-identification defender system DF. This system DF may be arranged at the data source, such as at the memory MEM where the data is held, or may be otherwise arranged, at a place where the data is generated, such as at the imager IA, or elsewhere/other data source. The re-identification defender DF may be understood as a go-in-between component, which is arranged and/or operative anywhere between data generator/source IA or data storage MEM on the one hand, and parties A, CS who wish to access the data.

The defender DF, for such it will be referred to herein for the sake of brevity, may act prospectively or retrospectively. In the prospective option, the original data X', once obtained, is processed by the defender DF before there is a request for this data. The processed data is then stored and is available for a requester. Each data item X' for different patients for example may be so processed in series or in parallel. In the retrospective option, the defender DF is operable only once a request for data, for example by attacker, comes in. Thus, the defender may include an event handler functionality to intercept such data request. The defender then processes the data, and it is the so processed data $X^a$ that is then made available for the requester CS, A, instead of the original data X'. In either case, prospective or retrospective, the defender operates to modify data X', as will be explained in more detail below. The original data X' is thus (slightly) modified, and it only this modified data $X^a$ that is held in memory/storage, etc MEM accessible to requester CS, A, in particular attacker A. The original data X' itself may not be made available, such as by encryption, keeping same offline, etc. Thus, whilst the legitimate user CS is usually not thought to have malicious intentions, they are also not given access to the original data X', but it is preferably only the modified data $X^a$ that is so made accessible. This affords a further layer or security, as the original data X' may still be leaked, inadvertently, even by the legitimate user CS.

As will be explained in more detail below, the modified data $X^a$ as produced by the defender DF, still allows legitimate use thereof, for example by the data consumer CS, such as a medical user, who wishes to process patient data X for medical purposes. Alas, the modified data $X^a$ is constructed so that it leads, with high likelihood, the re-identification ("re-ID")-attack by attacker awry. In distinction, the legitimate user may still obtain the same results when processing the modified data $X^a$, as they had obtained, had user CS been allowed to process the original data X'. But the attacker A will be left with a wrong re-identification result when processing the modified $X^a$, as opposed to the correct re-ID result, if attacker had been allowed access to the original (unmodified) data X'.

For example, legitimate use by the medical user may include the computing system CS processing the accessed (modified) data $X^a$ by a suitably configured (target) machine learning model M for domain knowledge purposes, such as in the medical realm. For example, the legitimate system CS may use the machine learning model M to establish, based on patient imagery $X^a$, whether a certain disease is present, such as in mammography screening, whether a certain treatment is likely to be successful, or in order to segment such imagery, or indeed to establish any other result in support of a medical task, or other task, whichever the domain at issue. Thus, even the legitimate user is only allowed to operate on the modified data $X^a$ produced by the defender DF. But, as explored below in more detail, the so modified imagery $X^a$ is modified in a specific way that does not compromise accuracy and operation of the ML model M used by legitimate user/system CS, whilst it does interfere with, and compromises results of, the attacker A's re-identifier model M* .

Both, attacker A and the legitimate user CS system, may each use respective, differently configured, machine learning models, such as in deep learning or any other, to processes the data X for their purposes. Deep learning models are a specific kind of artificial neural network("NN") type models. In NN models, computational nodes with weights are arranged in layers, starting from input layer proceeding through one or more hidden layers, and resulting in an output layer. In deep learning models, there is a plurality of such hidden layers which has been found to lead to good performance. Some NNs are convolutional, thus includes one or more convolutional layers that implements convolutional operators. Such CNNs (convolutional NNs) are advantageous for processing spatially correlated data, such as imagery. The layer may include different types of layers such as the said convolutional layer or pooling layers, etc. The multiple layers in DL models may preferably include multiple convolutional layers.

Data, such as modified image data X, is processed at input layer, passes as intermediate data (also referred to as feature maps) through the one or more hidden layers, and is then produced at output layer as an output result. Such result may include, in case of the legitimate user CS, a sought medical result, such as a prediction/estimate. Such prediction/estimate may include a classification result or a regression result. Thus, the result may indicate presence of a medical condition or prediction of an outcome for a radiotherapy treatment plan, a segmented image with the input image X segmented for certain regions of interest such as tissue, organ, organ part, tumor, etc. In case of the adverse user model M*, geared for re-identification attack the result is a re-ID result, such as a probability/score whether the processed image does or does not belong to a set of known individuals (sometimes referred to as the "gallery" in re-identification technology). Both the attacker and/or the legitimate (e.g., medical) user CS system may use machine learning models of the neural network types, or the two may use different ML models. Gradient-based models are preferred herein. The models M, M* are generally different in make-up and purpose as will be explained below in more detail.

In either case, the respective model M, M* has been trained previously on suitable training data. In such training, weights or other parameters of the models M, M* are adjusted, based on the training data. The training is controlled by an objective function, such a loss or utility function. Any training algorithm may be used, but preferably gradient based. For example, back-propagation techniques may be used. The training data may use a supervised learning setup. In setups, the training data may include input training data, some or each associated with a respective target ("label"). The objective function measures how well the model maps the training input data to its respective target. Parameters are adjusted in one or more iterations for example in the iterative scheme in order to improve the objective function. Once a pre-defined quality threshold is reached, the respective model is considered trained and can be used in deployment, such as for the intended medial task purposes by the medical consumer CS, or for re-ID attack purposes by the attacker A. Again, completely different types of models may be used by an attacker A and the legitimate user CS. Also, different types and kinds of models and/or training schemes may be used by each party CS, A. Non-supervised or semi-supervised training methods are not excluded herein.

As schematically indicated in Fig. 1, Attacker A and/or legitimate user CS may each include respective user interfaces UI, UI* which allows manipulating the received data, for example for processing by respective machine learning model M, M* as explained above. Attacker or legitimate user may wish to have the imagery and/or the result of their respective machine learning processing displayed on a respective display device DD.

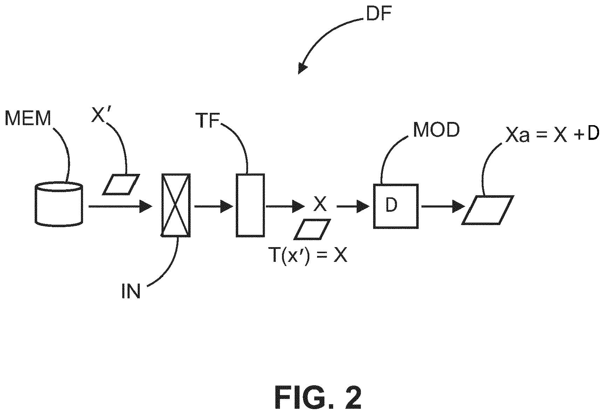

Reference is now made to the block diagram of Fig. 2, which provides more details on operation and components of the defender DF. As broadly explained above, the defender DF is configured herein to defend against re-identification attacks by an attacker A, using the suitably configured machine learning M*. Some or all of the original data X' is retrieved from memory MEM and received at input port IN. In some cases, operation on the original (raw) data X' as such may not be advisable, so here is a transformer or mapper component TF used, in order to first transform the original data X' to a suitable data representation first, and this is the processed by the defender DF. Thus, transformer TF may instantiate a mapping that maps the original data X' to the transformed data X=T(X'). However, such a transformer TF may be optional, as in some cases operation on the original data X' may be indeed done. Operation of the transformer TF in some instances is such that it produces a lower dimensional ("dim") representation of the original data X'. Such transformer may be thought to effect an embedding of the data A as points in a suitably structured and dimensioned data space. Such transformation TF may itself be referred to as "embedding". Such transformation, or re-representation of the data to the processed down the line, may allow faster and more memory efficient processing. Any one of a number of dimensional reduction techniques, such as principal components, or other may be used. In fact, the representation transformer TF may itself be arranged as a machine learning model such as an auto-encoder ("AE"), or a variational encoder ("VAE") as needed. In the earlier case, the auto-encoder is trained so that the original data X' is applied to the model which is then transformed to a lower dimensional representation. Such lower dimensional representation is then re-transformed back (hence auto-encoder) into the original input data. For example, the lower dimensional data X may be obtained as a latent representation within the auto-encoder's mode architecture. For example, the AE may be arranged as an NN. The lower representation may be accessed as a feature map in one of the hidden layers of the auto-encoder such as the one located centrally between input and output layer. The auto-encoder may itself be trained by a separate objective function, different from the objective function used for training the model M, M*. As said, use of such any such or other transformer TF may be optional, Thus, in such cases, the transformer may be thought of as the identity operator, when such transformer is not in fact used.

In some cases, the transformer TF may be part of the adverse model M*, such as may be integral thereto. Thus, the re-ID functionality of adverse model M* and the described transformer TF may be essentially coalesced into a single model, performing both functions. The model may be understood as the concatenation M* ∘ TF. But, again, neither adverse model M* nor target model M may use any transformer TF. Indeed, if such transformation is used at all, it is preferred that such transformer TF is used together with the adverse model M*, whilst target model M does not use such transformer TF and operates instead on the original data X' instead. Thus, any reference herein to the data X processable herein by defender DF includes a reference the original data X' as this may be processable instead. Thus, in each occurrence herein of the transformer TF, this may be taken as the identity operator. Thus, no such transformer is in effect used in such embodiments.

In the following we will refer to the data processed by data defender as "X", irrespective of whether this is the original X' or transformed data X=T(X'), unless such distinction is needed. This shorthand allows disencumbering notation, but is also conceptually justified as in most cases the transformer TF maps uniquely into data points so is configured (at least) as an injective operation. Thus, different data items X' for different individuals x is mapped to different embedded points X=T(X').

The input data, embedded/transformed or not, is modified by modifier MOD to produce the modified data $X^a$, such as modified imagery, which is then made available for access by any party, such as the legitimate user or the attacker, for processing, such as ML-processing by models M, M*.

As will be explained in more detail below, the modification operation by modifier MOD is so geared that it preserves the operational accuracy of the legitimate model M, whilst it distorts or otherwise interferes, at least with high probability, output of the attacker model M*. It is of note that if the transformer TF is used, its operation is different and in addition to the operation of the modifier MOD. Thus, the transformer TF merely allows a more efficient representation of the data A and such transformed data does not have the property (referred to below as "partial invariance") of the modified data $X^a$ as produced by the modifier MOD. Thus, the transformed data $T(X)=X$ may not allow defending against re-identification attacks as compared to the modified data $X^a$ as produced herein by operation of the modifier MOD. The modifier MOD may apply its modification operation component-wise for example per pixel or other component of the data. For example, in case the input data $X, T(X)$ is an image, the modifier MOD may operate to per-pixel on the input data image $X$ or $T(X)$. Not all pixels /component of a data item $X, T(X)$ may need to be so modified. Modification of a subset may suffice. Some or all components /pixels may be modified in the same manner, e.g. by the same amount ε, or some or each such components /pixels is modified differently. For example, such modification may include adding and/or multiplying a pixel with a number. Any other algebraic operation may be used. In generally and preferably, the modified data $X^a$ is, least for the human user, barely, or not, distinguishable from the input image/data X by the average human user. But even if it is, the modification is generally chosen to as to preserve aspects of global and local patterns. The right amount of modification that is not visually perceptible by most users may be found by tests run with a selection of the target audience (e.g., Radiologists, etc). The modification level/magnitude ε may be chosen in line with certain noise characteristics of the data source IA, explained in more detail below.

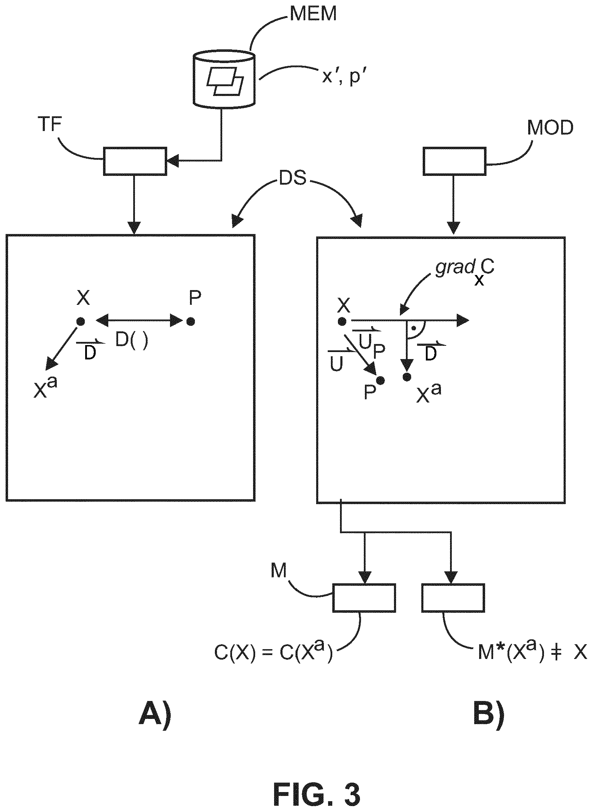

Reference is now made to Figs 3A, B which illustrate in yet more detail operation of the modifier MOD. Whilst reference to transformer TF is made, again, this is optional: Thus, transformer TF may be may conceptualized as the identity operator, and thus the below and above is of equal application in case no transformer is in fact used, thus when operation of modifier MOD on the original data is preferred.

Different data items X' and P', for example from different individuals x, p, may be processed by the transformer TF mapped into a data space DS such as a vector space with a suitable norm, or metric more generally, to yield elements X, P as illustrated in Fig. 3A. In an affine space DS, such elements may be visualized as data points as illustrated in Fig. 3A. Preferably, such transformer TF may be part of the adverse model M* as mentioned above, if such transformer is indeed used. Preferably, no such transformer is used for target model M. But may be used by adverse model M*. Data space DS may be Euclidean space, with dimension $m \times n$, with $m$ and $n$ being numbers of rows and columns of data X, such as when this is an image X. However, data may be any data, such as reports or other, Thus, the herein-described in not confined to image data, although this is indeed envisaged in the main.

A distance function $D()$ may be defined in data space DS that allows measuring how similar or close those data points (and hence the underlying data X',P', and hence by further extension the individuals x,p) are. Thus, the metric or distance function $D()$ is configured so that if the data pertains to the same individual, such data in general will yield a small distance, ideally zero, and different individuals get mapped to points in the data space DS, with a distance as measured by $D()$ that is larger than the threshold, but in any case, non-zero. Euclidean distance $D()$ for example may be used, in any required dimension. Thus, the distance measure D may be understood as a similarity measure as it measures the similarity of the mapped data points X, P. Thus, the distance function may itself be understood as a mapping from points of data space into the non-negative reals.

The modifier applies a modification $\Delta$ to the data which is shown only for X to yield the data $X^a$ that protects against adverse re-identification attacks. Such an operation may be understood as a vector operation, such as "shifting" of data point X:

$$X^a = X + \Delta \qquad\qquad (1)$$

The re-ID defence as afforded by defender DF is thus that the assumed known model M of the legitimate user still yields the same results for $X^a = X + \Delta$, as it would for the unmodified data X. Alas, the attacker's re-identification model M* will be led awry by yielding different results for the modified data X° as compared to the original data X. This is shown in the lower right of Fig. 3B. This re-ID defence conferring property may be referred to herein as "partial invariance". Thus, invariance in respect of results is preserved for the legitimate model M, whilst such invariance is not preserved for the re-ID model M*. This partial invariance is achieved by configuring the modification vector $\Delta$ in a specific manner. This is also illustrated in Fig. 3B, to which reference is now made in more detail. Any model, such as legitimate model M of consumer system CS or attacker A's model M* may be understood as a mapping of data X to the respective result, such as regression or classification. Specifically, in case of target model M, this may be a medical result. In case of adverse model M*, the result is different from the results M delivers, in particular M*'s output may be the re-DI result. Thus, each model may give rise to a model function, or predictor function C, R, such as $C: X \rightarrow M(X), R: X \rightarrow M(X)$. Each such function C,R may have a gradient grad (C) /grad (R), sometimes written herein without the brackets to disencumber notation.

Specifically, the gradient of predictor function C is illustrated in Fig. 3B as having a particular direction in the data space DS. It has been found herein that by choosing the modification vector $\Delta$ to be running in a different direction than the gradient of the legitimate predictor function C may yield modified data $X^a$ that has the partial invariance property. Specifically, it has been found that modifier vector $\Delta, X^a = X + \Delta,$ may be chosen to be orthogonal to the gradient grad (C) of function C. However, any other direction, as long as it is not along grad(C) (parallel or anti-parallel), may also be used. As will be explained in more detail below, a geometrical construction mechanism to construct orthogonal modification vectors $\Delta$ may be used. A randomized mechanism suitably constrained may be used instead of such a geometrical construction mechanism to yield suitable modification vectors $\Delta$. As envisaged herein, to be "not orthogonal" to gradient C may include herein being parallel and/or anti-parallel to gradient grad(C).

In some embodiments of construction, the modifier vector $\Delta$ may be understood in terms of a perturbation $U_P$ towards a chosen point P as shown schematically in Figs 3A, B. The point P in data space DS may represent another individual (e.g., patient p other than patient x, who is represented by data X instead).

The perturbation vector to such other point P may be projected onto the gradient of C at point X, and then the difference vector of the projection along gradient C and the perturbation vector $U_P$ is taken to yield modifier vector $\Delta$, now guaranteed to be orthogonal to gradient. Points X, P may be understood to form an associative pair with is completely arbitrary. Such pairs may be established automatically by system through a randomizer in the set of all patients {x} or whichever set of data is to be processed for re-ID hardening by defender DF. Optionally, a user interface UI may be used where user can interactively establish such pairs. Later, when processing point P accordingly, computing time can be saved by simply taking $\Delta(P) = -\Delta(X)$. Thus, for the other pair member, in this case P in the associative pair (X,P), the modifier vector may be chosen as the negative (anti-parallel) of the modifier vector $\Delta(X)$ earlier computed for the member X processed first. Data point/vector P may be chosen at random. In some use cases, P may be chosen to have some similarity to X as measured by a distance function $D()$.

In some cases, no further knowledge of the adverse model M* may be needed, as some models are ill-conditioned, so a small modification is enough to compromise their results as in re-ID model M*, whilst the proposed gradient based approach ensures, at least with sufficient likelihood, the desired invariance of known model M against such modification/perturbation $\Delta$. Indeed, as illustrated by Goodfellow et al (in "Explaining and Harnessing Adversarial Examples", available online at arXiv service, reference code arXiv:1412.6572v3 [stat.ML], published 2015), even an added human-imperceptibly small perturbation may mislead a classifier to misclassify a slightly perturbed image of a Panda (Ailuropoda melanoleuca) as one of a Gibbon (Hylobatidae), although the classification was correct if no such perturbation is applied. See page 3, Fig. 1 in said cited Goodfellow et al.

It will be understood herein that for each point X that is to be modified, the gradient grad (C) will need to be evaluated at position X, $grad (C) = grad_X(C)$. However, publicly available knowledge, or indeed leaked knowledge, may be used to estimate re-ID action of adverse model M*. A prototype model M** may be build and trained on data X, in order to so approximate or estimate re-ID action of the adverse model M*. In some embodiments, such knowledge may be used herein to estimate $X^a$. In particular, a gradient of model M* may be used by modifier MOD, in addition to grad C of model M. In some embodiments or any of the embodiments herein, the gradient of grad D° M* ≈ grad D° M** may be used by modifier MOD.

A specific, vector-geometry based construction of such modifier vector Δ is now described in more detail. For example, a projection-based modification construction may be used. More specifically, a Gram-Schmidt-type vector-based, or other similar modification, may be used in embodiments. The construction may be described based on the perturbation $U_P$ towards random or predetermined point P. P may vary or may remain fixed, as needed. Varying P may be preferred in some embodiments, but fixed P constructions (for some or all X) may be done instead in some contexts.

Specially, Given the target model M (such as with deep learning (DL) architecture or any other), configured/-trained for image classification C(X), it is desirable that a similar response for $X^a$ and X is obtained. Therefore, what is proposed herein is not to use the projection onto a vector that is orthogonal to the gradient of C with respect to X. Since the gradient of C with respect to X is orthogonal to the level set of the classification function C, such a change will minimize the effect of the perturbation:

$$X^a = X + \varDelta, \ \varDelta = \epsilon \cdot \left( U - \frac{G^T U}{G^T G} G \right) \tag{2}$$

where "$T$" is a transpose operator, $U$ any vector $U$ in data space DS, not parallel/anti-parallel to G. In some embodiments, $U$ may be chosen as the said perturbation vector $U_P$. That is, in some embodiments, $U_P$ is the perturbation towards the other data point $P$ (e.g., an image of patient p) as introduced above. G represents the gradient of the classification function C of the image X as per target model M:-

$$G = \frac{\partial C(X)}{\partial X} \tag{3}$$

As said, in (2), and other vector $U$ not parallel/anti-parallel to $G$ may be used instead of perturbation $U_P$. Choosing $U = U_P$ being more one example, which, however, has been found to yield good results and allows a canonical, straightforward implementation. It may have the further benefit that such configuration $U = U_P$ may better "mislead" M* by leading to a misclassification of X into P. This may conceal to A that the modified data X is actually misleading its model *. However, in the general case, perturbation U may not need to point to another such point P that represents an existing patient $P$.

In some embodiments, effect of the distance function $D()$ on the output of M* may be accounted for in the above computing of modified data $X^a$. Thus, in some embodiments, good results can be obtained by choosing/configuring the distance function so that distances between points X, P are magnified. Other distance functions with such magnifying property can be use instead. In embodiments, in order to account for unwanted effects of the distance function D in data space DS, and to avoid undue response by the predictor function C, in some embodiments, effects of the distance function D on changes in C are included in the construction of the modifier Δ. Thus, the effect on how much the re-ID model M* "spreads" its outputs can be taken into account, when defining :-

$$U = U_P = \text{sign}\left(\frac{\partial M^* \circ D(P,X)}{\partial X}\right) \tag{4}$$

Thus, perturbation $U_P$ as per (4) may be used above in eqs (2)(3) in some embodiments. However, the construction based on orthogonal shifts relative to *grad C* is of general application, and any vector U may be used to compute its projection along grad C and take the difference of said vector U by its projection along grad C to obtain orthogonal modifier Δ.

In some embodiments still, model M* is essentially a distance function D and provides as ID results distance values D(X). In thus instances, model M* may be identified with the distance function D, and the following vector may be used instead for perturbation $U_P$:

$$U = U_P = \text{sign}\left(\frac{D(P,X)}{\partial X}\right) \tag{5}$$

In eqs (2)-(5), instead of M*, if this is not known, the above-described approximation/remodelling M** may be used instead.

In any one the above and below, if model M* uses transformer TF (such as embeddings), the gradient *grade D° M** ° TF) ≈ grad (D ° M* ° TF)* may be used for determining Δ.

Although the proposed projection-based construction such as at any one of eqs (2)-(5) has been found to yield good results and affords efficient processing, it is still to be construed herein as merely one example from many, and such construction does not limit the general principles of what is disclosed herein. Specifically, geometrical vector constructions, other than projection based, are also envisaged herein in different embodiments. What is more, the proposed principles are not confined to geometrical constructions, as others, such as statistical, etc are also envisaged. In particular, the construction in terms of perturbation $U_P$ and pairs (X,P) is, whilst efficient, not limiting herein.

In the above, the "signum" operator *sign()* indicates the direction of the unit vector whilst ε includes a suitably small amount of perturbation ε that can be found by trial and error as briefly mentioned above, e.g. by conducting image perception tests on the target audience. The perturbation amount ε may be applied vector-component-wise, as need be. Construction of the direction sign ensures that the modification Δ is in fact orthogonal to gradient. Modifications that are substantially orthogonal but may not necessarily be exactly orthogonal have been shown to still yield good results and are preferred herein.

The results of applying the perturbation ε to image data may be imperceptible to the average user, and can be objectively established by conducting the said perception test using test imagery. The perturbation may be equal to or lower than the noise level in the data X acquiring detector equipment used. The noise level can be measured by experiment on phantoms for example. The noise level may be readily available from detector manufacturer's data specification sheets for the particular make and model of detector that was used to obtain the original imagery X'. The perturbation ε may be random. It may differ from image to image, or, may be reused from image to image, as needed. Such reuse strategy may allow yet more responsive implementation which may be useful in Cloud/client-server settings, etc.

In eqs(1)-(5), the " + " operation is a vector operation, such as addition. It may be applied component-wise. As the data X may be an image, having rows and columns, a representation as a matrix may be envisaged, and the same for the shift vector Δ as matrx of same size as X. Thus, eqs(1)-(5) may be implemented as component-wise matrix addition, with elements $X$, $\Delta$ and $X^a$ treated as elements of vector space DS. Applying the modification by operations other than addition may also be envisaged in some instances so the operation "+" may indeed by a vector addition, but may also relate to other combination operation. If transformer TF is used, dimensional correspondence may need to be ensured, such as by back-transformation, padding, etc.

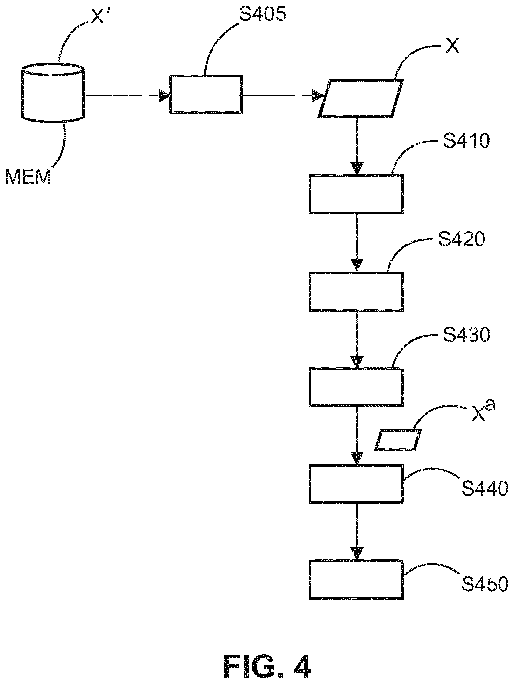

Reference is now made to the flow chart of Fig. 4, which illustrates steps of a computer implemented method for defending original data X' against identification attacks as envisaged herein in embodiments. The proposed method is not necessarily tied to the system architectures as shown above, but the said below method may be understood to constitute a teaching in its own right.

At step S405, original data X' is accessed.

At an optional step S410, the original data and may be transformed into a representation more suitable for down-stream processing, as such as lower dimensional representation. Dimension reduction techniques may be used. The transformation may be implemented as an embedding into a data space (such as vector space) as points or vector. The transforming (operation) may be machine learning based. For example, an auto-encoder may be used to derive latent representations in terms of feature maps or any other. However, this transformation into a lower dimensional, or a different, otherwise more suitable, representation is optional. Thus, down-stream operation on the original data instead of on transformed data, is also envisaged herein. Preferably, such transformation TF is not used for the target model M, but may be used in the adverse model M* as embeddings for example. However, this too is optional.

At step S420 the data to be re-ID defended is received.

At step S430 the received data is modified, to yield the re-identification-attack-hardened, modified, data $X^a$ which is then made available at step S440 for access by any party to process, legitimate or adverse.

In more detail and in some embodiments, at step S450, the data $X^a$ may be processed by a target machine learning model M, in order to obtain a regression or classification result, or any other result in an applicable domain, such as medical or other. The processing S450 may further include, in addition, or instead of, such domain processing by target ML model M, adverse processing by a (different) machine learning model M* for re-ID purposes. Thus, if the processed data X is personal data, the adverse processing by adverse model M* may wish to seek a result to confirm that the data X belongs to a certain specific individual (a person) known to the attacker.

The modification at step S430 ensures that the modified data $X^a$ produced has the partial invariance property as explained above, or the modification at step S430 ensures at least helps to achieve this with sufficient likelihood. Thus, the modification results in modified data Xa that preserve results obtainable by the legitimate/target domain model M, irrespective of whether original or modified data is processed, but at the same time such data modified data $X^a$ compromises operation of the adverse model M* by causing such adverse model M* to produce different results compared to when processing the original, unmodified, data X. Thus, the legitimate user can go about processing even the modified data, as before with the original data, with difference (hence invariance) in results obtained, whilst the re-identification attacker is more likely to obtain wrong results. Thus, for example, the attacker may not be able to identify any person in the gallery using mode M*, or model M* may in fact yield a response that indicates another person as a "hit", instead of the person to whom the data X actually pertains.

In embodiments, the modification operation S430 is based on any one of eqs (1)-(5) or similar, or on any other data modifier approach to ensure that the modification Δ is not along the gradient of target model M's predictor function C.

Thus, modification in a vector data space, in which the data X is presentable, is along a vector direction other than the gradient of the predictor function C of the target model M at point X. In embodiments, the direction is orthogonal, or is approximately (within some negligible error margin) to such gradient *grad C()*. A construction of modification S430 based on associative points and/or in terms of perturbation vector $U_P$ to a different point P as mentioned above is envisaged in some embodiments. In embodiments, the orthogonal construction considers the effect of the distance function D. Thus, in some embodiments, the construction of Δ is (further) based on the gradients of *grad* R() and/or *grad* D(), in particular may be based on *grad* D(X,P), for example as described above at eqs (4),(5).

The above method step S430 may be repeated for each data item X that needs to be hardened against re-ID attacks. The method may be practiced in prospective or retrospective fashion, as needed.

The components of system SYS, in particular or defender DF, may be implemented as one or more software modules, run on one or more general-purpose processing units PU such as a workstation associated with the imager IA, or any other computing device, such as laptop, smartphone, etc.

Alternatively, some or all components of the defender DF may be arranged in hardware such as a suitably programmed microcontroller or microprocessor, such an FPGA (field-programmable-gate-array) or as a hardwired IC chip, an application specific integrated circuitry (ASIC), integrated into the imaging system IA. In a further embodiment still, the defender DF may be implemented in both, partly in software and partly in hardware.

The different components of the defender DF may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope. Such reference signs may be comprised of numbers, of letters or any of alphanumeric combination.

**Claims**

1. A system (SYS) for processing of data, comprising:

   an interface (IN) capable to receive input data based on personal data processable by a target machine learning model (M) for producing a result; and
   a modifier (MOD) capable of modifying the input data into modified input data, such that the said result corresponds to a result obtainable by having the modified input data processed by the target machine learning model (M), whilst the modified data is still capable of defending against a re-identification attack.

2. The system of any one of the preceding claims, wherein the modifier is capable of so modifying the input data based on the gradient of target model (M).

3. The system of any one of the preceding claims, wherein the modifier is capable of so modifying along a direction other than a direction of the said gradient of target model (M).

4. The system of claim 3, wherein the modifier is capable of so modifying along a direction that is essentially orthogonal to the direction of the said gradient of model (M).

5. The system of any one of the preceding claims, the re-identification attack performable by a further machine leaning model (M*).

6. The system of claim 5, wherein the modification is based on a gradient of said further model (M*).

7. The system of any one of the preceding claims 5 and 6, wherein the modification is based on a gradient of a distance function, where such distance function measures the distance between the results obtainable by the further machine leaning model (M*).

8. The system of any one of the preceding claims, wherein the said result corresponds to a domain task, such as a medical task.

9. The system of any one of the preceding claims, wherein at least one of the target model (M) and the further model (M*) is based on an artificial neural network.

10. The system of any one of the preceding claims, wherein the personal data includes patient data.

11. The system of any one of the preceding claims, wherein the personal data includes image data.

12. An arrangement (IAR) including the said system (SYS) of any one of the preceding claims, and one or more of i) one or more memories (MEM) on which is stored the input data, ii) the target model (M), iii) the further model (M*).

13. Method of data processing, comprising:

   receiving (S420) input data based on personal data, processable by a target machine learning model (M) for producing a result; and
   modifying (S430) the input data into modified input data, such that the said result corresponds to a result obtainable by having the modified input data processed by the target machine learning model (M), whilst the modified data is still capable of defending against a re-identification attack.

14. At least one computer program element, which, when being executed by at least one processing system (SYS), is adapted to cause the processing system (SYS) to perform the method as per claim 13.

15. At least one computer readable medium (MEM) having stored thereon the at least one program element of claim 14, and/or on which is stored the target model or the further model, or an approximation of the said further model.

**FIG. 1**

**FIG. 2**

**FIG. 3**

**FIG. 4**

## EUROPEAN SEARCH REPORT

**Application Number**

EP 23 20 0974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUN ZONGKUN ET AL: "Differential Privacy for Data and Model Publishing of Medical Data", IEEE ACCESS, vol. 7, 14 October 2019 (2019-10-14), pages 152103-152114, XP011752330, DOI: 10.1109/ACCESS.2019.2947295 [retrieved on 2019-10-24] * page 152103, left-hand column, paragraph 1 - right-hand column, paragraph 2 * * page 152104, right-hand column, paragraph 1; figure 1 * * abstract * * page 152108, left-hand column, paragraph 5 * * Algorithm 1; page 152108, right-hand column * * page 152109, right-hand column, paragraph 2 - paragraph 3 * | 1-14 | INV. G06F21/62 G06N3/0455 G06N3/0464 G16H10/60 G16H30/00 G06N3/084 G06N3/094 |
| A | KIM BACH NGOC ET AL: "Privacy-Net: An Adversarial Approach for Identity-Obfuscated Segmentation of Medical Images", IEEE TRANSACTIONS ON MEDICAL IMAGING, IEEE, USA, vol. 40, no. 7, 12 March 2021 (2021-03-12), pages 1737-1749, XP011863627, ISSN: 0278-0062, DOI: 10.1109/TMI.2021.3065727 [retrieved on 2021-06-30] * page 1739, right-hand column, last paragraph - page 1741, right-hand column, paragraph 2 * | 1-15 | TECHNICAL FIELDS SEARCHED (IPC) G06F G06N G16H |

-/--

The present search report has been drawn up for all claims

| Place of search | Date of completion of the search | Examiner |
|---|---|---|
| Munich | 26 March 2024 | Herri, Edmond |

Europäisches
Patentamt

European
Patent Office

Office européen
des brevets

## EUROPEAN SEARCH REPORT

Application Number

EP 23 20 0974

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KRALL ALEXANDER ET AL: "Gradient Mechanism to Preserve Differential Privacy and Deter Against Model Inversion Attacks in Healthcare Analytics", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 5714-5717, XP033816717, DOI: 10.1109/EMBC44109.2020.9176834 [retrieved on 2020-08-24] * page 5715, right-hand column, paragraph 5 - last paragraph * * page 5716, left-hand column, paragraph 1 - paragraph 2 * | 1-15 | |
| A | DINGFAN CHEN ET AL: "GS-WGAN: A Gradient-Sanitized Approach for Learning Differentially Private Generators", ARXIV.ORG, CORNELL UNIVERSITY LIBRARY, 201 OLIN LIBRARY CORNELL UNIVERSITY ITHACA, NY 14853, 15 June 2020 (2020-06-15), XP081696325, * page 3, paragraph 4 - page 5, paragraph 3; figure 1 * | 1-15 | TECHNICAL FIELDS SEARCHED (IPC) |
| A | KAI PACKH\"AUSER ET AL: "Deep Learning-based Anonymization of Chest Radiographs: A Utility-preserving Measure for Patient Privacy", ARXIV.ORG, CORNELL UNIVERSITY LIBRARY, 201 OLIN LIBRARY CORNELL UNIVERSITY ITHACA, NY 14853, 24 July 2023 (2023-07-24), XP091570814, * page 3, paragraph 1 - page 5, paragraph 3; figure 1 * | 1-15 | |

The present search report has been drawn up for all claims

| Place of search | Date of completion of the search | Examiner |
|---|---|---|
| Munich | 26 March 2024 | Herri, Edmond |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **K PACKHÄUSER et al.** Deep learning-based patient re-identification is able to exploit the biometric nature of medical chest X-ray data. *NATURE*, 2022, vol. 12, 14851

- **T. M. MITCHELL**. Machine Learning. McGraw-Hill, 1997, 2
- **GOODFELLOW et al.** Explaining and Harnessing Adversarial Examples. *arXiv service, reference code arXiv:1412.6572v3*, 2015